## Europäisches Patentamt
## European Patent Office
## Office européen des brevets

(19)

(11) Veröffentlichungsnummer: **0 284 569**
**A1**

(12)

# EUROPÄISCHE PATENTANMELDUNG

(21) Anmeldenummer: 88810200.1

(22) Anmeldetag: 25.03.88

(51) Int. Cl.⁴: **A 61 K 47/00**
A 61 K 9/48, A 61 K 9/70,
A 61 L 15/06, C 08 L 89/06

(30) Priorität: 27.03.87 CH 1173/87

(43) Veröffentlichungstag der Anmeldung:
28.09.88 Patentblatt 88/39

(84) Benannte Vertragsstaaten:
AT BE CH DE ES FR GB GR IT LI NL SE

(71) Anmelder: Luisi, Pier Luigi, Prof. Dr.
Moussonstrasse 22
CH-8044 Zürich (CH)

(72) Erfinder: Luisi, Pier Luigi, Prof. Dr.
Moussonstrasse 22
CH-8044 Zürich (CH)

(74) Vertreter: Blum, Rudolf Emil Ernst et al
c/o E. Blum & Co Patentanwälte Vorderberg 11
CH-8044 Zürich (CH)

(54) Blendpolymere.

(57) Die Blendpolymeren sind dadurch erhältlich, dass man wenigstens ein proteinhaltiges Material und wenigstens ein Polysaccharid enthaltendes Material in wenigstens einem Lösungsmittel suspendiert, diese Suspension so lange auf eine Temperatur bis zu 50° C erwärmt, bis sich wenigstens 50% der vorhandenen Menge an proteinhaltigem Material und Polysaccharid enthaltendem Material gelöst haben, dann das Gemisch auf Raumtemperatur abkühlt und das Lösungsmittel abdampft.

Diese Blendpolymeren können zur Herstellung von Formkörpern, insbesondere von Gebrauchsgegenständen, vorzugsweise biokompatible Gebrauchsgegenstände, insbesondere Behälter, Kapseln, Folien oder Filmen, verwendet werden.

EP 0 284 569 A1

**Beschreibung**

## Blendpolymere

Die vorliegende Erfindung bezieht sich auf Blendpolymere, auf Verfahren zu deren Herstellung, auf deren Verwendung zur Herstellung von Formkörpern, sowie auf Formkörper, die aus solchen Blendpolymeren hergestellt sind.

Gelatine ist ein wertvolles Biopolymer, welches viele technische Anwendungen gefunden hat, z.B. in der Lebensmittelindustrie, in der Photographie, in der Verpackungsindustrie und in der Pharmakologie. Die Verarbeitung ist eine wichtige Voraussetzung für seine Anwendbarkeit, d.h. die Verarbeitung muss einfach und billig sein und gleichzeitig die Herstellung von ganz verschiedenen Produkten erlauben. Von besonderem Interesse sind diejenigen Verarbeitungsmethoden, die es erlauben, Gelatine mit anderen Komponenten zu mischen: damit kann man Materialien mit neuen Eigenschaften erzeugen, obwohl das Hauptelement noch Gelatine ist. Um Gelatine zu schmelzen, muss diese normalerweise auf Temperaturen über 150° C, gewöhnlich zwischen 170° C und 190° C, erwärmt werden. Bei diesen hohen Temperaturen werden aber viele mögliche Additive durch Wärmeeinwirkung zerstört. Eine solche Erwärmung benötigt zusätzlich eine beträchtliche Zufuhr an Energie, was selbstverständlich mit hohen Kosten verbunden ist. Gelatine kann aber auch in Wasser gelöst werden. Aber auch das Verdampfen von Wasser aus einer wässrigen Gelatinelösung benötigt eine beträchtliche Zufuhr an Energie, und ist demzufolge auch nicht billig.

In diesem Patentgesuch werden u.a. zwei neue Methoden beschrieben, die es erlauben, auf einfache Weise vorzugsweise Gelatine mit verschiedenen Komponenten zu mischen. Diese Materialien sind besonders geeignet, um Filme, Kapseln, Folien, Gefässe und andere biokompatible Gegenstände herzustellen.

Die Hauptidee der Erfindung besteht darin, zwei polymerische Komponenten (z.B. Gelatine und Stärke) in Anwesenheit eines dritten Stoffes zu mischen, welcher die Fähigkeit hat, die zwei Polymere chemisch zu einander zu binden, z.B. durch Wasserstoffbrücken. Polymere wie Stärke und Gelatine besitzen schon Gruppen, mit welchen sie im Prinzip miteinander in Wechselwirkung eintreten können; sie sind aber zu steif und chemisch nicht homogen, um wirklich chemisch zu interagieren. Der intermediäre Stoff dient als Kollante zwischen den zwei Polymeren, indem er als Zwischenbrücke funktioniert. Als solche "Kollante" kommen dann in diesem Sinne Stoffe in Betracht, die per sé einfache H-Bruecken bilden, wie z.B. Harnstoff, Glyzerin, Propylenglykol, Ethylenoxid und andere polyvalente Alkohole, wie Polyethylenoxide oder Polyvinylalkohole, oder Amine oder Aminosäuren (z.B. Glyzin) wie auch Aldehyde, wie Formaldehyd, Paraformaldehyd und Glutaraldehyd, oder Säuren wie Ameisensäure, Phosphorsäure, Schwefelsäure.

Die Idee, ein Blendpolymer zwischen Stärke und Gelatine in Anwesenheit eines Kollantes herzustellen, ist nicht in der Literatur vorhanden. Es war aber bekannt, dass Glycerin an Gelatine addiert werden kann, was zu einer Verbesserung der Verarbeitung des Polymeren führt. Es sind auch einige Mischungen von Stärke und Gelatine bekannt. Zum Beispiel wurde der Effekt von Gelatine auf die Geltemperatur von Stärke untersucht (J. Djakovic et al., Technol. Mesa 1985, 148-153); es wurde ein Gelatine-Stärke-Schwamm für medizinische Zwecke vorgeschlagen (Prusyczynski et al., Polim. Med. (1977, 7, 9-17), und andere Autoren haben den Effekt von Gelatin Bloom Nummer auf die physikalische Eigenschaften von Lactose-Stärke Tabletten untersucht (Georgakopoulos P.P. und Malamataris S., Pharm. Ind. 1976, 38, 728-732). Coazervaten von Gelatine und chemisch modifizierte Stärke wurden auch hergestellt als innere Wand von Kapseln (T. Hayashi und andere, Japan. Kokai, 1976, 10, 181). Die Koazervation von Stärke mit Gelatine in Anwesenheit von Chloral-hydrat wurde von H.Y. Chung und M.M. MacMasters untersucht (Stärke, 18, 1377-82, 1966); diese Studien waren aber auf Lösungen begrenzt und nicht auf die Eigenschaften der festen Polymere. Es wurde auch der Einfluss von Stärke und Lactose auf die Abgabegeschwindigkeit von Arzneimitteln aus Gelatinekapseln untersucht (J.E. Davies, und J.T. Fell, J. Pharm. Pharmacol. 1973, 25, 431-52). Es gibt Berichte über Stärke-extended Gelatineprodukte (Szymanski C.D. und G.J. Heimstetter, DE-OS 2 335 509, 1974 US Appl. 272,395, 17. Juli 1972). Schliesslich, wurde der Einfluss von Stärke auf die Disintegration von Hart-Gelatine-Kapseln (P. De Beukelaer et al., Drug Dev. Ind. Pharm. 1985, 11, 431) oder auf Filmen für die Lebensmittelindustrie-Verpackungen (Mitsubishi Acetate Co., Ltd., Jpn. Kokai Tokkyo Koho JP 60 76, 336 (85 76, 336) untersucht.

Die Besonderheit der Erfindung liegt auch darin, dass die Polymermasse auf einer sehr einfache Weise verarbeitet werden kann, und zwar mit allen üblichen Methoden, die für Polymerschmelzen oder Hochvisko semassen gebraucht werden, wie z.B. Spritzgiessen, Kalandieren, Filmcasting, Extrusion, usw.

Der Vorteil der neuen Methode liegt auch darin, dass während der Bildung der bevorzugten Gelatinepolymere verschiedenartige spezielle Verbindungen zugefügt werden können, z.B. Salze, Pigmente, schwere Metalle, Füllstoffe sowie Enzyme und Bakterienzellen, so dass Materialien für ganz besondere Zwecke, z.B. für Biotechnologie, vorbereitet werden können.

Die Erfindung ist durch die Merkmale in den unabhängigen Ansprüchen gekennzeichnet.

Bevorzugte Ausführungsformen sind in den abhängigen Ansprüchen definiert.

Zusammenfassend liegt die Bedeutung der Erfindung darin:

- Es wird eine neue, schnelle und billige Methode für die Verarbeitung von vorzugsweise Gelatine, besonders für die Herstellung von biokompatiblen Polymerfilmen und Membranen oder Kapseln für pharmazeutische Anwendungen, beschrieben;

- das bevorzugte Gelatine kann auf eine sehr einfache Weise mit verschiedenartigen Verbindungen gemischt werden, was die Steuerung der Eigenschaften der Endprodukte erlaubt; und

- Enzyme und Bakterien können in den bevorzugten Gelatinepolymeren inkorporiert werden.

In diesem Patent werden zwei Methoden beschrieben, um insbesondere Gelatine neu zu verarbeiten, die im folgenden 1. Methode und 2. Methode genannt werden.

## 1. Methode

Herstellung von Gelatinefilmen aus Mikroemulsionsgelen.

Es wurde vor kurzem in den oben erwähnten Literaturstellen eine Methode beschrieben, um Gele aus Wasser-in-Oel-Mikroemulsionen (oder Umkehrmizellen) erhalten zu können. Das Verfahren kann folgendermassen zusammengefasst werden: eine gewisse Konzentration (z.B. 150 mM) eines Tensides, welches in der Lage ist, Umkehrmizellen zu bilden (z.B. Aerosol-OT, abgekürzt AOT), wird in einem Kohlenwasserstoff (z.B. Isooktan) gelöst. Zu dieser Lösung wird eine konzentrierte Gelatine-Lösung in Wasser zugefügt (z.B. 10% v:v einer 40% Gelatine-Lösung in Wasser); die Mischung wird erwärmt bis ca. 50° C, und beim Abkühlen unter +30° C wird das ganze System ein Gel. Diese Mikroemulsionsgele sind mit verschiedenen physikalischen Methoden charakterisiert worden und intensive Untersuchungen sind im Gange, um die Struktur diesr neuen Materialien zu klären, und den Anwendungsbereich in verschiedenen Gebieten der Pharmazie und Physik zu erforschen.

Der nächstliegende Stand der Technik ist in der schweizerischen Patentanmeldung Nr. 4983/84-9 und der Europäischen Patentanmeldung Nr. 85 904 955.3 beschrieben.

Wir haben jetzt zu unserer Ueberraschung festgestellt, dass sowohl Wasser als auch das Lösungsmittel (z.B. Isooktan) aus diesen Gelpräparaten quantitativ entfernt werden können, und dass daraus Polymerpräparate, insbesondere Filme, resultieren, die hauptsächlich aus Gelatine und AOT bestehen.

Das Verfahren kann anhand folgender vier Schritte beschrieben werden:

## 1.1) Gelbildung:

Es wird zuerst ein Mikroemulsionsgel hergestellt. Als Lösungsmittel sind z.B. tiefsiedende Paraffine, wie Pentan, Hexan, Cyclohexan, geeignet; es kann sogar ein Gas wie Butan benutzt werden, die unter hohem Druck verflüssigt werden; oder chlorierte Kohlenwasserstoffe, oder deren Mischungen.

## 1.2) Zusatzstoffe:

Entweder vor der Bildung des Gels, oder wenn das Gel schon gebildet ist, werden Stoffe zugefügt. Dieser Schritt war auch schon in der Literatur beschrieben; der Unterschied besteht aber darin, dass spezielle Stoffe zugefügt werden, die man in Gelatinefilme für spezielle Anwendungen braucht. Solche Stoffe sind z.B. anorganische Salze, wie $ZnCl_2$ LiCl, oder organische Stoffe wie Glycerin und seine Mono- oder Di- oder Triester; oder Formamid, Methylformamid, und andere Stickstoff enthaltende Niedermolekulargewichtsverbindungen; oder Biopolymere wie Polysaccharide, z.B. Heparin, Chondroitinsulfate, Stärke, Chitine, Zellulose; schliesslich auch synthetische Polymere, wie Polyisobutylen, Polystyrol, und andere, die direkt löslich in der öligen Phase sind, wie auch Polymere, wie Polyvinylalkohol, die in der wässrigen Phase solubilisiert werden können. Steinmehl und Riess können auch als Zusatzstoffe wirken. Auch Enzyme, Bakterien und Zellenteilchen, wie Mithocondria, können beigefügt werden. Der Gehalt dieser zugefügten Stoffe kann zwischen 0,001 und 60 Gew.-% liegen, insbesondere zwischen 1 und 10 Gew.-%.

## 1.3) Formgebung

Das so vorbereitete Gel wird im dritten Schritt des Verfahrens verarbeitet, z.B. im Falle der Herstellung von Folien wird das Gelmaterial auf eine Platte gegossen. In der früheren Literatur hat man eben nicht realisiert, dass die so gewonnenen Gele direkt verarbeitet werden können, um Folien, Kapseln oder andere Gegenstände herzustellen, die dann durch den nachfolgenden Schritt 1.4) in mechanisch stabile und preisgünstige Stückwerke verarbeitet werden können. Die Dicke des Filmes kann zwischen 0,01 mm und 5 cm, insbesonders zwischen 1 und 5 mm, sein.

## 1.4) Trocknung

Diese Operation besteht darin, dass das organische Lösungsmittel vom Gelmaterial entfernt wird. Am einfachsten kann dies durch Verdampfung bei Raumtemperatur gemacht werden. Die Verdampfung kann beschleunigt werden, z.B. mit einem Warmluftgerät; oder indem man die Form in einem Ofen erwärmt. Die Art und Weise hängt von der Flüchtigkeit des Lösungsmittels ab, z.B. mit Gelen aus Butan, Pentan und Hexan ist die Raumtemperaturverdampfung in wenigen Stunden vollständig.

Nach dem Abdampfen des Lösungsmittels entstehen Filme oder Produkte, deren chemische Analyse gezeigt hat, dass sie aus Gelatine und Tensid bestehen (Wasser dampft zusammen mit dem organischen Lösungsmittel ab). Eine X-ray Analyse der Filme zeigt, dass das Material amorph ist.

Die so gewonnenen Filme wurden auch als Membrane für die Trennung von Biomaterialien untersucht. Zum Beispiel wurde untersucht, ob ein Film, gewonnen mit 150 mM AOT und 10% Gelatine, Peptide durchlassen kann, die in Chloroform gelöst sind (5 mM Konzentration des Peptides). Es wurde festgestellt, dass nach drei Stunden 17% Z-Phenylalanyl-methylester und 24% BOC-Tyrosin durchgeflossen waren.

Die Permeabilität der Filme für Gase, z.B. Helium und Stickstoff, wurde auch untersucht (aus einem Gel mit 10% Gelatine, 150 mM AOT, 14% Wasser und Isooktan), und es wurde festgestellt, dass das Material eine gewisse Selektivität zeigt, z.B. die Permeabilität von Helium war 1,1, die von Stickstoff 0,2 $cc/cm^2 \cdot cm \cdot s \cdot bar \cdot 10^7$.

Beispiel 1.1

Es werden 10 ml einer 150 mM AOT Isooktan-Lösung bei Raumtemperatur vorbereitet, zu wlecher 1,4 ml einer wässrigen Lösung, die 1 g Gelatine (Blum n.250) enthält, zugefügt wird. Diese Suspension wird bei 50° C erwärmt, wobei sie klar wird. Beim Abkühlen bis auf Raumtemperatur entsteht ein Gel, welches auf eine metallische Platte gegossen wird. Nach ca. 5 Stunden entsteht ein fester Film, welcher hart und durchsichtig ist.

Beispiel 1.2

Wie oben, aber die wässrige Lösung enthält, zusammen mit Gelatine, $ZnCl_2$, 20 mM overall Konzentration. Das resultierende Gel ist nicht durchsichtig; der Film, welcher daraus entsteht, ist hart und durchsichtig.

Beispiel 1.3

Wie oben, aber mit 20 mM $Na_2SO_4$ anstatt $ZnCl_2$. Aehnliche Eigenschaften beim Gel und beim Film wie oben.

Beispiel 1.4

Wie oben, aber Tween 85 wird anstatt Salz zugefügt und zwar in einer Konzentration von 1,5 Gew.-%. Das resultierende Gel ist durchsichtig, der Film ist hart aber wasserlöslich.

Andere Beispiele können später beigefügt werden: mit Pigmenten, mit Enzymen, mit Bakterien, mit Condroitinsulfat, mit DMFA und DMSO; mit Polyisobutylen und Polyvinylalkohol.

2. Blendpolymere von Gelatine und Stärke

Das zweite Verfahren, um Polymerblende aus vorzugsweise Gelatine zu machen, braucht kein Tensid und keine Mikroemulsionslösung. Beispielsweise werden Gelatine und Stärke direkt gemischt, vorzugsweise mit Hilfe eines Kollantes, und es resultiert ein Blendpolymer, zu welchem weitere Zusätze, nach Bedarf, zugefügt werden können (z.B. Pigmente oder Biomoleküle).

Das Verfahren kann beispielhaft folgendermassen beschrieben werden:

1) Gelatine und Stärke werden in einem Gefäss zusammen gemischt. Der relative Gehalt an Stärke kann zwischen 0,5 und 90 Gew.-% sein, insbesondere zwischen 20 und 60 Gew.-%. Dann wird Wasser zugefügt, so dass das gesamte Gewicht von Gelatine und Stärke zwischen 0,5 und 70% des insgesamten Gewichtes ist, insbesondere zwischen 2,5 und 40%. Die Suspension wird unter Rühren in einem Wasserbad bis auf eine Temperatur von 50° C erhitzt, und zwar so lange, bis Gelatine gelöst ist.

2) Glycerin wird zugefügt, und zwar in einem Gewichtsanteil von 10% bis 300% des Stärkeanteils, insbesondere 30 bis 100%. Die Temperatur wird auf 90° C gebracht, womit die Lösung sehr viskos wird. Wird die Temperatur langsam auf Raumtemperatur gebracht, dann bildet sich ein Gel.

Diese ersten beiden Schritte können auch in einem einzigen Arbeitsgang gemacht werden.

3) Die Lösung ist sehr homogen. Wenn damit z.B. Kapseln hergestellt werden (siehe Beispiel) zeigt Scanning Electron Mikroskopie (Hitachi S700) kein Zeichen von Heterogenität. Preliminare Untersuchungen mit Schmelzpunktmikroskopie zeigen, dass die Kapseln weich werden und bei 150° C schrumpfen.

Der Wassergehalt im Endprodukt (z.B. in Kapseln) kann leicht bestimmt werden, indem die Fertigteile in einem Vakuumofen bei 60° C gehalten werden und der Gewichtsverlust gemessen wird.

Die so gewonnene Lösung ist stabil. Mit Lyophilisation (freeze-drying) enthält man ein weisses Polymer, welches gute mechanische Eigenschaften besitzt und z.B. geschmeidig und mechanisch fest wie auch nicht brüchig ist.

2.1. Beispiel für die Herstellung von Kapseln

5 g Weizenstärke werden mit 8 g Gelatine in 130 ml Wasser gemischt. Im zweiten Schritt (siehe oben) werden 5 g Glycerin zugefügt. Die Temperatur der Lösung wird bei 40 - 50° C max. gehalten und es werden damit Kapseln hergestellt. Dafür wird die im Tiefkühlschrank auf -20° C abgekühlte "molding bar", welche mit Silikonfett leicht geschmiert wurde, in die Lösung eingeführt, langsam herausgezogen und bei Raumtemperatur getrocknet. Wenn das Material trocken ist, können die Kapseln von den metallischen Fingern abgezogen werden. Die Dichte der Kapseln kann leicht kontrolliert werden, indem man die Konzentration der Lösung und die Temperatur (und damit die Viskosität der Lösung) steuert. Der Wassergehalt der trockenen Kapseln teträgt 0,4 Gew.-%.

2.2. Beispiele für die Herstellung von Filmen

Allgemeines Vorgehen

Bei sämtlichen Beispielen kann folgendermassen vorgegangen werden:

Alle Substanzen werden kalt so lange gemischt, bis die Lösung keine Klumpen mehr aufweist. Anschliessend erhitzt man langsam auf 90° C und hält die Lösung unter Rühren so lange bei dieser Temperatur bis sie homogen, transparent und viskos ist.

Anschliessend kann sie verarbeitet werden.

Beispiele für Blendpolymere Stärke und Gelatine mit Hilfe verschiedener Komponenten

2.2.1. Glycin

Beispiel:

3 g Weizenstärke, 1,8 g Gelatine, 1 g Glycin und 50 ml Wasser ergeben ein homogenes, leicht transparentes, leicht sprödes Polymer.

2.2.2. Harnstoff

Beispiel:

3 g Weizenstärke, 1,8 g Gelatine, 0,9 g Harnstoff und 50 ml Wasser ergeben ein leicht sprödes Polymer.

### 2.2.3. Aethylenglycol

Beispiel:

3 g Weizenstärke, 1,8 g Gelatine, 50 ml Wasser und 1 g Aethylenglycol ergeben ein leicht sprödes, sehr homogenes Polymer. Fügt man mehr Aethylen glycol zu, z.B. 2 g gibt es ein sehr elastisches, homogenes Polymer.

### 2.2.4. Polyvinylalkohol (15000)

Beispiel:

3 g Weizenstärke, 1,8 g Gelatine, 50 ml Wasser und 1 g Polyvinylalkohol ergeben ein sehr homogenes, leicht sprödes Polymer.

### 2.2.5. Glycerin

Beispiel:

2 g Weizenstärke, 2,8 g Gelatine, 45 ml Wasser und 0.9 g Glycerin ergeben ein homogenes, sehr elastisches Polymer.

Will man den Weizenstärke-Anteil erhöhen und weniger Gelatine verwenden, kann man mit grösserer Glycerinkonzentrationen die grosse Elastizität des Polymers beibehalten.

### 2.2.6. Blendpolymer mit Ketoprofene

Ausgangsmaterial: Weizenstärke    2 gr
     Gelatine Bloom 300     1.5 gr
     Glycerine     1.5 gr
     Ketoprofene (Carlo Erba)     350 mg/0.5 ml EtOH
     (Batch AM 16 B041)

2 gr. Stärke, 1.5 gr. Gelatine und 1.5 gr. Glycerin werden bei Raumtemperatur in 60 ml $H_2O$ unter Rühren suspendiert. Anschliessend wird das Gemisch auf 90° C erhitzt und 15 Min. lang bei dieser Temperatur unter Rühren gehalten. Das nun homogene Gemisch wird unter starkem Rühren auf 40° C runterthermostatiert. Sobald die Temperatur des Gemisches 40° C erreicht hat, werden 350 mg Ketoprofene (gelöst in 0.5 ml EtOH) unter starkem Rühren zugegeben. Nach ca. 2 Min. wird das Gemisch in Polystyrolschalen 0.2 ml/cm² dick ausgegossen und bei Raumtemperatur an der Luft trocknen gelassen.

### 2.2.7. Blendpolymer-Pulver mit Ketoprofene
Gleiche Herstellung wie oben, aber ohne Glycerin.
Der fertige Film ist spröde und wird mit Trokkeneis in Reibschale pulverisiert.

## Patentansprüche

1. Blendpolymere, dadurch erhältlich, dass man wenigstens ein proteinhaltiges Material und wenigstens ein Polysaccharid enthaltendes Material in wenigstens einem Lösungsmittel suspendiert , diese Suspension so lange auf eine Temperatur bis zu 50° C erwärmt, bis sich wenigstens 50% der vorhandenen Menge an proteinhaltigem Material gelöst haben, dann das Gemisch auf Raumtemperatur abkühlt und das Lösungsmittel abdampft.

2. Blendpolymere nach Anspruch 1, dadurch erhältlich, dass man dem Gemisch vor dem Abkühlen auf Raumtemperatur noch wenigstens einen Hilfsstoff, vorzugsweise eine mindestens bifunktionelle, Wasserstoffbrückenbildende Verbindung, wie z.B. ein polyvalenter Alkohol, vorzugsweise Aethylenglykol, Propylenglykol, oder Harnstoff, Ethylenoxid, Polyethylenoxide, Polyvinylalkohole, Amine, Aminosäure, vorzugsweise Glycin, Aldehyde, vorzugsweise Formaldehyd, Paraformaldehyd, Glutaraldehyd, Carbonsäuren, vorzugsweise Ameisensäure, Phosphorsäure, Schwefelsäure, Pyrophosphat, Glycerin oder einen seiner Mono-, Di- oder Triester, und/oder wenigstens ein Pigment, und/oder wenigstens ein biologisch aktives Material, insbesondere ein Biopolymer, vorzugsweise Enzyme, Bakterien, Nukleinsäuren, Zellen, Mithochontrien, Plasmide, und/oder wenigstens ein anorganisches Salz, beispielsweise $ZnCl_2$, $LiCl_2$, beimischt.

3. Blendpolymere nach einem der Ansprüche 1 bis 2, dadurch gekennzeichnet, dass das proteinhaltige Material ein natürliches proteinhaltiges Material ist, insbesondere ein Mehl, ein Zellextrakt tierischen oder pflanzlichen Ursprungs, Gelatine, Elastin oder Keratin.

4. Blendpolymere nach einem der Ansprüche 1 bis 3, dadurch gekennzeichnet, dass das Polysaccharid enthaltende Material ausgewählt ist aus Stärke, Amylose, Heparin, Chitinen, Chondroitinsulfaten und Polyglykanen.

5. Blendpolymere nach einem der Ansprüche 1 bis 4, dadurch gekennzeichnet, dass das Lösungsmittel ausgewählt ist aus Wasser, Dioxan, Dimethylsulfoxid, Formamid oder einem seiner substituierten und/oder alkylierten Derivate, beispielsweise Methylformamid oder Dimethylformamid.

6. Blendpolymere nach einem der Ansprüche 1 bis 5, dadurch gekennzeichnet, dass das proteinhaltige Material und das ein Polysaccharid enthaltende Material in einer Menge von 0,5 bis 70 Gew.-%, insbesondere 2,5 bis 40 Gew.-%, bezogen auf das gesamte Gewicht, vorhanden sind.

7. Blendpolymere nach einem der Ansprüche 1 bis 6, dadurch gekennzeichnet, dass der in Anspruch 2 erwähnte Hilfsstoff in einer Menge von 10 bis 300 Gew.-%, insbesondere 30 bis 100 Gew.-%, bezogen auf das vorhandene Gewicht des ein Polysaccharid enthaltenden Materials, vorhanden ist.

8. Blendpolymere nach einem der Ansprüche 1 bis 7, dadurch gekennzeichnet, dass das in Anspruch 2 erwähnte Pigment, und/oder das biologisch aktive Material und/oder das anorganische Salz in einer Menge von 0,001 bis 60 Gew.-%, insbesondere 1 bis 10 Gew.-%, bezogen auf das gesamte Gewicht, vorhanden sind (ist).

9. Verfahren zur Herstellung von Blendpolymeren nach Ansprüchen 1 und 2, dadurch gekennzeichnet, dass man wenigstens ein proteinhaltiges Material und wenigstens ein Polysaccharid enthaltendes Material in wenigstens einem Lösungsmittel suspendiert, diese Suspension so lange auf eine Temperatur bis zu 50° C erwärmt, bis sich wenigstens 50% der vorhandenen Menge an proteinhaltigem Material, gegebenenfalls zusammen mit den in Anspruch 2 erwähnten Stoffen, gelöst haben, dann das Gemisch auf Raumtemperatur abkühlt und das Lösungsmittel abdampft.

10. Verfahren nach Anspruch 9, dadurch gekennzeichnet, dass man ein Blendpolymer nach einem der Ansprüche 2 bis 8 herstellt.

11. Verwendung der Blendpolymere nach einem der Ansprüche 1 bis 8, zur Herstellung von Formkörpern, und zwar vorzugsweise mit den Verarbeitungsmethoden, die man bei der Verarbeitung von Polymerschmelzen anwendet, insbesondere Formpressen und Filmgiessen.

12. Verwendung nach Anspruch 11, dadurch gekennzeichnet, dass der Formkörper ein Gebrauchsgegenstand, vorzugsweise ein biokompatibler Gebrauchsgegenstand, insbesondere ein Behälter, eine Kapsel, eine Folie oder ein Film, ist.

13. Formkörper, hergestellt aus einem Blendpolymer gemäss einem der Ansprüche 1 bis 8.

14. Formkörper nach Anspruch 13, dadurch gekennzeichnet, dass er ein Gebrauchsgegenstand, vorzugsweise ein biokompatibler Gebrauchsgegenstand, insbesondere ein Behälter, eine Kapsel, eine Folie oder ein Film, ist.

15. Verfahren zur Herstellung von Blendpolymeren mittels
- Auflösen von wenigstens einem proteinhaltigen Material in möglichst wenig Wasser,
- Auflösen von wenigstens einem Tensid in wenigstens einem organischen Lösungsmittel,
- Vermischen dieser beiden Lösungen, und
- Umwandlung dieses Gemisches in ein Mikroemulsionsgel,
dadurch gekennzeichnet, dass man aus dem genannten Gel das Wasser und/oder das organische Lösungsmittel abdampft.

16. Verfahren nach Anspruch 15, dadurch gekenn zeichnet, dass man vor der Gelbildung noch wenigstens einen Zusatzstoff, vorzugsweise wenigstens ein Pigment, und/oder wenigstens ein Arzneimittel, und/oder wenigstens ein biologisch aktives Material, insbesondere ein Biopolymer, vorzugsweise Enzyme, Bakterien, Nukleinsäuren, Zellen, Mithochontrien, Plasmide, und/oder wenigstens ein anorganisches Salz, beispielsweise $ZnCl_2$, $LiCl_2$, und/oder wenigstens ein Co-Tensid, beimischt.

17. Verfahren nach einem der Ansprüche 15 bis 16, dadurch gekennzeichnet, dass das proteinhaltige Material ein natürliches proteinhaltiges Material ist, insbesondere ein Mehl, ein Zellextrakt tierischen oder pflanzlichen Ursprungs, Gelatine, Elastin oder Keratin.

18. Verfahren nach einem der Ansprüche 15 bis 17, dadurch gekennzeichnet, dass das Tensid ausgewählt ist aus dem Tensid AOT, Natrium-1,2-bis(2-Ethylhexyloxycarbonyl)-1-ethansulfonat, Benzalconiumchlorid, CTAB (Cethyltrimethyl-ammoniumbromid), Tensiden auf der Grundlage von anderen quaternären Ammoniumsalzen, Tensiden auf der Grundlage von Ethylenglykol, Tensiden natürlicher Herkunft, wie Phospholipide, Lecithine, Phospholecithine, Phosphodilcoline, synthetischen Tensiden, die als Grundkern Glycerin haben, und Tensiden, die die Struktur von Estern von Fettsäuren, wie z.B. Sorbitantristearat oder Polyoxyethylensorbitan Oleat (Polisorbato 80), aufweisen.

19. Verfahren nach einem der Ansprüche 15 bis 18, dadurch gekennzeichnet, dass das organische Lösungsmittel ausgewählt ist aus natürlichen organischen Lösungsmitteln, wie Squalan, essentiellen Oelen, Terpenen, Pflanzenölen, Miglyol, Estern von natürlichen Fettsäuren, wie z.B. Palmitinsäure, Stearinsäure usw., perfluorierten Kohlenwasserstoffen und Kohlenwasserstoffen, wie z.B. Octan, Isooctan, Pentan, Hexan, Cyclohexan.

20. Verwendung der gemäss dem Verfahren nach einem der Ansprüche 15 bis 19 hergestellten Blendpolymeren zur Herstellung von Formkörpern, und zwar vorzugsweise mit den Verarbeitungsmethoden, die man bei der Verarbeitung von Polymerschmelzen anwendet, insbesondere Formpressen und Filmgiessen.

21. Verwendung nach Anspruch 20, dadurch gekennzeichnet, dass der Formkörper ein Gebrauchsgegenstand, vorzugsweise ein biokompatibler Gebrauchsgegenstand, insbesondere ein Behälter, eine Kapsel, eine Folie oder ein Film, ist.

22. Formkörper, hergestellt aus einem Blendpolymer, erhalten gemäss dem Verfahren nach einem der Ansprüche 15 bis 19.

23. Formkörper nach Anspruch 22, dadurch gekennzeichnet, dass er ein Gebrauchsgegenstand, vorzugsweise ein biokompatibler Gebrauchsgegenstand, insbesondere ein Behälter, eine Kapsel, eine Folie oder ein Film, ist.

| | | | |
|---|---|---|---|

<table>
<tr><td colspan="4"><strong>EUROPÄISCHER RECHERCHENBERICHT</strong></td></tr>
</table>

**Europäisches Patentamt**

**EUROPÄISCHER RECHERCHENBERICHT**

Nummer der Anmeldung

EP 88 81 0200

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 701 034 (SCHEVER GmbH) <br> * Seite 5, Zeile 28 - Seite 7, Zeile 23; Seite 10, Beispiel 1 * <br> --- | 1-14 | A 61 K 47/00 <br> A 61 K 9/48 <br> A 61 K 9/70 <br> A 61 L 15/06 <br> C 08 L 89/06 |
| P,X | EP-A-0 219 762 (DESITIN ARZNEIMITTEL GmbH) <br> * Spalten 9-11, Beispiel * <br> --- | 1-14 | |
| X | DE-A-2 552 126 (SUMITOMO BAKELITE CO.) <br> * Seite 9, Zeile 26 - Seite 22, Zeile 21; Seite 25, Zeile 21 - Seite 32, Ende * <br> --- | 1-14 | |
| X | FR-A-2 571 253 (NIPPON KAYAKU K.K. et al.) <br> * Seiten 8,9, Beispiele 1-3 * <br> --- | 1-14 | |
| X | DE-A-2 815 153 (HERMANSSON) <br> * Insgesamt * <br> --- | 1-14 | |
| D,A | WO-A-8 602 264 (LUISI) <br> * Seite 17, Zeile 1 - Seite 22, Ende * <br> ----- | 15-23 | RECHERCHIERTE SACHGEBIETE (Int. Cl.4) <br><br> A 61 K <br> A 61 L <br> C 08 L |

Der vorliegende Recherchenbericht wurde für alle Patentansprüche erstellt

| Recherchenort | Abschlußdatum der Recherche | Prüfer |
|---|---|---|
| DEN HAAG | 16-06-1988 | BENZ K.F. |

EPO FORM 1503 03.82 (P0403)